# EUROPEAN PATENT APPLICATION

(11) **EP 3 858 357 A1**
(43) Date of publication of application: **04.08.2021**
(21) Application number: 20305070.3
(22) Date of filing: 28.01.2020
(51) Int. Cl.: A61K 31/496, A61K 45/06, A61P 37/04, A61P 31/12, A61P 35/00

(54) **USE OF AZOLE COMPOUNDS TO STIMULATE THE IMMUNE SYSTEM AND AS INHIBITORS FOR S-PLA2GIB**

(71) Applicant: Diaccurate, 75008 Paris (FR)
(72) Inventor: THEZE, Jacques, 75007 PARIS (FR)
(74) Representative: Cabinet Becker et Associés

(57) **Abstract**

The present invention relates to compositions and methods to induce or stimulate an immune response in a subject using particular azole compounds. More specifically, the invention concerns the use of the azole-based compositions for inhibiting an enzymatic activity of sPLA2 and for treating disorders associated with an immune deficiency. The invention is particularly effective in the treatment of infectious diseases, immune disorders, or cancers.

## Description

### FIELD OF THE INVENTION

The present invention relates to compositions and methods to induce or stimulate an immune response in a subject using particular azole compounds. More specifically, the invention concerns the use of the azole-based compositions for inhibiting an enzymatic activity of sPLA2 and for treating disorders associated with an immune deficiency. The invention is particularly effective in the treatment of infectious diseases, immune disorders, or cancers.

### BACKGROUND OF THE INVENTION

Secretory phospholipases A2 (sPLA2) are esterases that hydrolyze the sn-2 ester of glycerophospholipids and constitute one of the largest families of lipid hydrolyzing enzymes (Lambeau & Gelb 2008). In addition to the well-established functions of one of these enzymes in digestion of dietary phospholipids and another in host defense against bacterial infections, accumulating evidence shows that some of these sPLA2 are involved in arachidonic acid release from cellular phospholipids for the biosynthesis of eicosanoids, especially during inflammation, or in promoting atherosclerosis and cancer. Thus, sPLA2 may have functions related to binding to cellular target proteins in a manner independent of their lipolytic enzymatic activity.

The inventors have previously characterized that sPLA2 of group IB (sPLA2GIB) is a key endogenous factor of the immune response (WO2015/097140). More particularly, the inventors have demonstrated that sPLA2GIB plays a crucial role in the mechanism underlying the unresponsiveness of CD4 T cells observed in various pathological conditions such as infectious diseases, cancer or immune disorders. It was thus proposed and documented by the inventors that sPLA2GIB modulators are effective for stimulating the immune system in a subject, especially for the treatment of diseases associated with an immune deficiency. Examples of such sPLA2GIB modulators are disclosed for instance in WO2015/097140, WO2017/037041 or WO2017/060405.

The invention stems from the finding of a novel class of sPLA2 inhibitors. More particularly, the inventors have identified a particular group of azole-based compounds that exhibit potent sPLA2GIB inhibitory effect and thus represent valuable agents for stimulating an immune response.

### SUMMARY OF THE INVENTION

An object of the invention relates to a method for stimulating or inducing an immune response in a subject, comprising exposing the subject to a compound of formula A, or a salt, ester, hydrate, racemate, enantiomer, prodrug or metabolite thereof.

A further object of the invention relates to a method of treatment of an immunodeficiency disorder in a subject, comprising exposing the subject to a compound of formula A, or a salt, esther, hydrate, racemate, enantiomer, prodrug or metabolite thereof.

A further object of the invention relates to a method of treatment of a viral infection and/or associated disease in a subject, comprising exposing the subject to a compound of formula A, or a salt, esther, hydrate, racemate, enantiomer, prodrug or metabolite thereof.

A further object of the invention relates to a method of treatment of a bacterial infection and/or associated disease in a subject, comprising exposing the subject to a compound of formula A, or a salt, esther, hydrate, racemate, enantiomer, prodrug or metabolite thereof.

A further object of the invention relates to a method of treatment of a cancer in a subject, comprising exposing the subject to a compound of formula A, or a salt, esther, hydrate, racemate, enantiomer, prodrug or metabolite thereof.

A more particular embodiment of the invention relates to a method of treating AIDS in a HIV-infected subject, comprising administering to the subject a compound of formula A or a salt, ester, hydrate, racemate, enantiomer, prodrug or metabolite thereof.

Another object of the invention relates to the use of a compound of formula A, or a salt, ester, hydrate, racemate, enantiomer, prodrug or metabolite thereof, for the manufacture of a medicament for inducing or stimulating an immune response, or for treating an immunodeficiency disorder in a subject.

Another object of the invention relates to a compound of formula A, or a salt, ester, hydrate, racemate, enantiomer, prodrug or metabolite thereof, for use in a method of inducing or stimulating an immune response, or of treating an immunodeficiency disorder in a subject.

Another object of the invention relates to a compound of formula A, or a salt, ester, hydrate, racemate, enantiomer, prodrug or metabolite thereof, for use for treating a viral or bacterial infection, or an associated disorder, in a subject.

Another object of the invention relates to a compound of formula A, or a salt, ester, hydrate, racemate, enantiomer, prodrug or metabolite thereof, for use for treating cancer in a subject.

The compound of formula A, or the salt, ester, hydrate, racemate, enantiomer, prodrug or metabolite thereof, may be used alone. Preferably, the compound of formula A according to the invention, is a sPLA2GIB inhibitor.

In another aspect, the invention relates to the use of the compound of formula A in combination with another active ingredient, such as for instance another sPLA2GIB inhibitor.

In another aspect, the invention relates to a pharmaceutical composition comprising (i) a compound of formula A, or a salt, ester, hydrate, racemate, enantiomer, prodrug or metabolite thereof, and (ii) a further antiviral or anticancer agent, for combined, separate, or sequential administration.

In another aspect, the invention relates to a pharmaceutical composition comprising: (i) a compound of formula A, or a salt, ester, hydrate, racemate, enantiomer, prodrug or metabolite thereof, and (ii) another sPLA2GIB inhibitor, preferably selected from the group consisting of antibodies or derivatives thereof, inhibitory nucleic acids, peptides, small drugs and soluble receptors, for combined, separate, or sequential administration.

The invention may be used in any mammalian subject, including humans and non-human animals such as, without limitation, domesticated animals (e.g., cows, sheep, cats, dogs, and horses), non-human primates (such as monkeys), rabbits, and rodents (e.g., mice and rats). It is particularly suited for use in human subjects.

### LEGENDS TO THE FIGURES

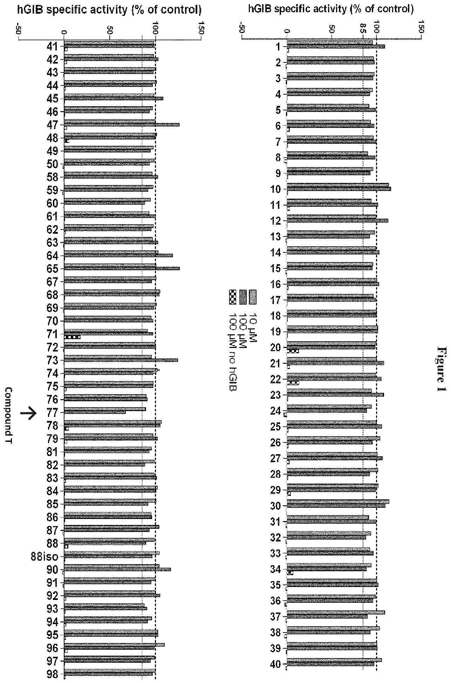

**Figure 1****:** Screening of the inhibition of sPLA2hGIB enzymatic activity by small molecules previously identified by *in silico* screening. 0% represents non-specific hydrolysis in the absence of sPLA2hGIB, and 100 % represents sPLA2hGIB activity with DMSO (1 %). Only the molecule #77 (Compound T, Terconazole) causes a decrease in sPLA2hGIB specific enzymatic activity greater than 15 % at 100 µM. Terconazole inhibits sPLA2hGIB by about 33 %.

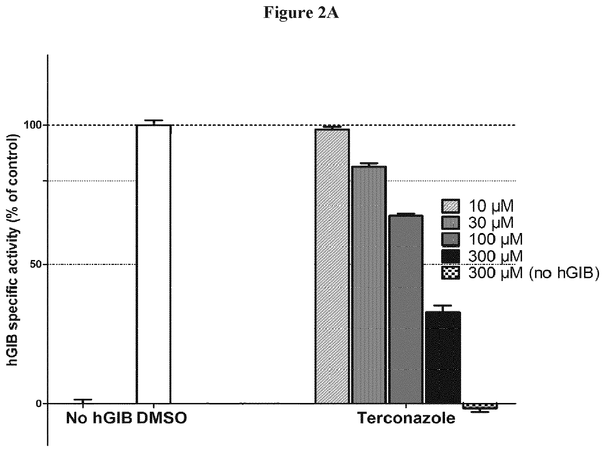

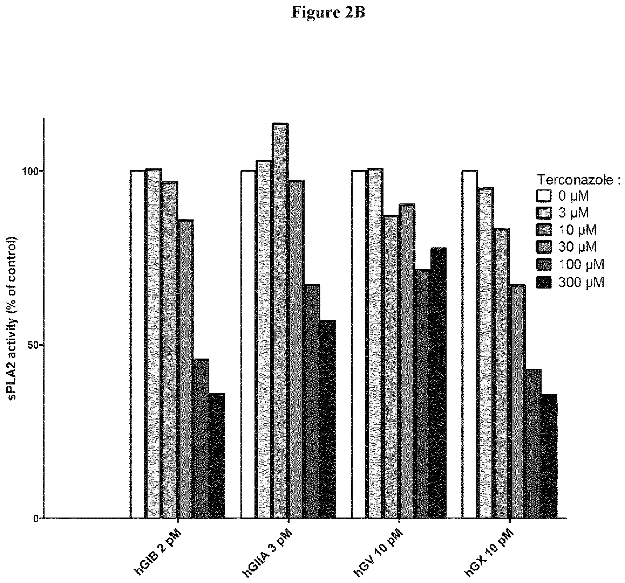

**Figure 2****: (A)** Inhibition of sPLA2hGIB (2 pM) specific enzymatic activity by compound T (Terconazole), using methods described in Rouault, Biochemistry, 2006*.* The condition 'no hGIB' (0%) represents the non-specific hydrolysis of phospholipids present in *E. coli* membranes radio-labeled with [³H]oleate. The condition 'DMSO' represents hGIB activity alone with DMSO 1 % (the solvent used to dissolve Terconazole). Phospholipase sPLA2hGIB is pre-incubated with different concentrations of Terconazole (PRESTWICK CHEMICAL, Illkirch-Graffenstaden); **(B)** Inhibition of the enzymatic activity of different sPLA2 (i.e., sPLA2hGIB, sPLA2hGIIA, sPLA2hGV, sPLA2hGX) by compound T (Terconazole) under conditions identical to Figure 2A.

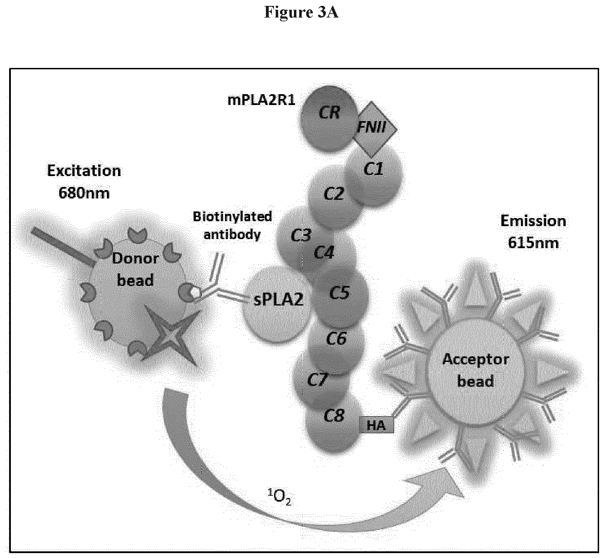

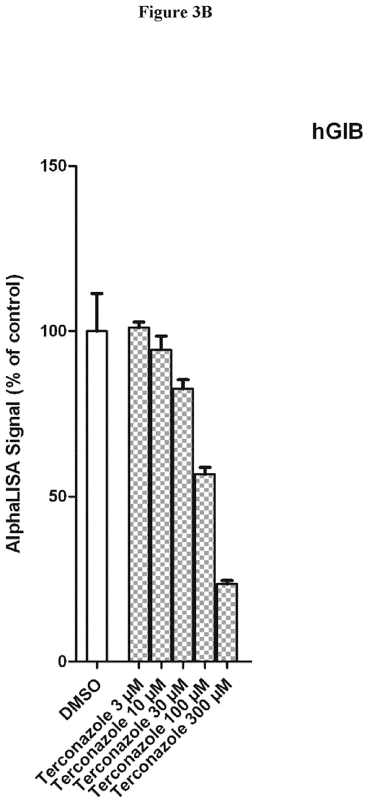

**Figure 3****: (A)** Schema of principle of the measurement of sPLA2: The sPLA2hGIB (or sPLA2hGIIF) binding to the whole extracellular part of HA-tagged mPLA2R1 is measured using AlphaLISA® technology. HA-tagged receptors are in the presence of sPLA2hGIB or sPLA2hGIIF, then in the presence of a specific biotinylated anti-sPLA2 antibody; "acceptor" beads are coupled to an anti-HA antibody (PerkinElmer) and "donor" beads are coupled to Streptavidine (PerkinElmer). The binding of a HA-tagged receptor and a sPLA2 in solution results in a proximity of donor and acceptor beads, allowing for energy transfer and the production of a light signal; **(B)** Competition of Terconazole with sPLA2hGIB binding to whole mPL2R1: the addition of compond T (Terconazole) competes in a dose-dependent manner for binding between mPLA2R1 receptor and sPLA2hGIB.

### DETAILED DESCRIPTION OF THE INVENTION

The present invention relates to compositions and methods for inducing or stimulating an immune response in a subject in need thereof. The invention more specifically provides particular azole compounds which can be used for the treatment of disorders associated with an immune deficiency such as infectious diseases, immune disorders or cancers.

The present invention shows the remarkable capacity of particular azole compounds to inhibit sPLA2GIB. Such compounds thus represent novel and potent agents for stimulating the immune system in human subjects, such as for inducing or stimulating CD4 T cells in a subject, and may thus be used to treat a variety of conditions.

An object of the invention thus relates to a compound of formula A, or a salt, ester, hydrate, racemate, enantiomer, prodrug or metabolite thereof, for use in inducing or stimulating an immune response, especially in a subject suffering from an immune disorder, an immunodeficiency disorder, an infectious disease (such as viral or bacterial disease) or a cancer; or for treating an immune disorder, an immunodeficiency disorder, an infectious disease (such as viral or bacterial disease) or a cancer, in a subject in need thereof.

A further object of the invention relates to a compound of formula A, or a salt, ester, hydrate, racemate, enantiomer, prodrug or metabolite thereof, for use in stimulating CD4 T cells in a subject.

Another object of the invention relates to the use of a compound of formula A, or a salt, ester, hydrate, racemate, enantiomer, prodrug or metabolite thereof, for the manufacture of a medicament for inducing or stimulating an immune response, in particular, in a subject suffering from an immune disorder, an immunodeficiency disorder, an infectious disease (such as viral or bacterial disease), or a cancer.

Another object of the invention relates to a method for stimulating or inducing an immune response in a subject, comprising exposing the subject to a compound of formula A, or a salt, ester, hydrate, racemate, enantiomer, prodrug or metabolite thereof.

Another object of the invention relates to a method of treatment of an immune disorder, an immunodeficiency disorder, infectious disease such as AIDS, or a cancer, in a subject in need thereof, comprising exposing the subject to a compound of formula A, or a salt, esther, hydrate, racemate, enantiomer, prodrug or metabolite thereof.

### Compounds of formula A

Compounds of formula A designate compounds having the following structure A: 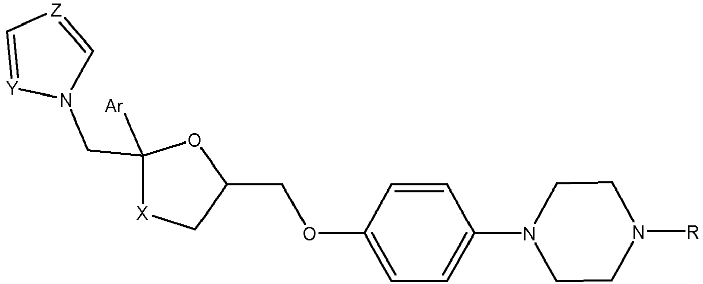 wherein:
- Ar is an aryl group, optionally substituted by one or more halogen atoms;
- X is CH2 or O (oxygen atom);
- Y and Z both are respectively:
   either N (nitrogen atom) and N (nitrogen atom),
   or CH or N (nitrogen atom);
- R is a -COCH3, -CH(CH3)2, 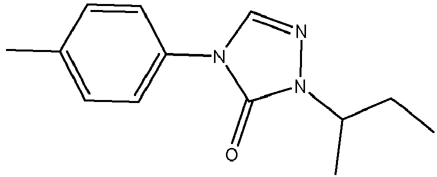 or 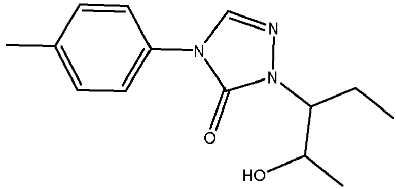 group;
   or a salt, ester, hydrate, racemate, enantiomer (e.g. R or S form), prodrug or metabolite thereof.

Compounds of formula A have been previously proposed for treating fungal infections disclosed in, e.g., WO96/29325. However, their ability to induce or stimulate the immune system has never been suggested. Moreover, the ability of said compounds to treat patients suffering from an immune disorder, an immunodeficiency disorder, a viral or bacterial infection, or a cancer, had never been suggested.

In a preferred embodiment, the componds for use in the invention are compounds of formula A, wherein Ar is a phenyl group substituted by one or two halogen atoms, preferably chlorine or fluorine.

In a further preferred embodiment, the compounds for use in the invention are compounds of formula A, wherein Y and Z are both nitrogen atoms.

In a further preferred embodiment, the compounds for use in the invention are compounds of formula A, wherein X is an oxygen atom.

Preferably, the compounds for use in the invention are compounds of formula A selected in the group consisting of terconazole, itraconazole, posaconazole or ketoconazole.

In a most preferred embodiment, the compound is 1-{[(2S,4S)-2-(2,4-dichlorophenyl)-4-{[p-(4-isopropyl-1-piperazinyl)phenoxy]methyl}-1,3-dioxolan-2-yl]methyl}-1H-1,2,4-azole (also designated 1-[4-[[2-(2,4-dichlorophenyl)-2-(1,2,4-triazol-1-ylmethyl)-1,3-dioxolan-4-yl]methoxy]phenyl]-4-propan-2-ylpiperazine) (compound T, Terconazole), or a pharmaceutically acceptable salt, hydrate, or prodrug thereof.

The structure of compound T is represented below:

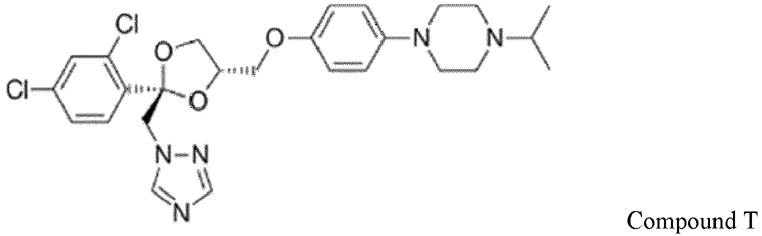

Compounds of formula A may be produced according to methods known *per se* in the art, as illustrated for instance in WO96/29325.

The term "salt" refers to any pharmaceutically acceptable inorganic or organic acid/basic addition salt of a compound of the present invention. Pharmaceutically acceptable acid salts according to the invention include, without limitation, salts of acetic acid, nitric acid, tartric acid, hydrochloric acid, sulfuric acid, phosphoric acid, methane sulfonic acid, camphor sulfonic acid, oxalic acid, maleic acid, succinic acid or citric acid. Pharmaceutically acceptable basic salts of the invention include, without limitation, sodium, potassium, calcium, magnesium, ammonium, or choline salts.

The term "prodrug" as used herein refers to any functional precursor of a compound of structure A which, when administered to a biological system, generates said compound as a result of e.g., spontaneous chemical, enzymatic, biological and/or metabolic reaction(s). Typical prodrugs have the structure Y-A wherein Y is a protective group and is cleaved from the prodrug *in vivo* to release compound A. Prodrugs are usually inactive or less active than the resulting drug and can be used, for example, to improve the physicochemical or pharmacokinetic properties of a compound.

The term "metabolite" designates a molecule which results from the *in vivo* modification or processing of a compound A after administration to an organism. Such modifications may occur through specialized enzymatic systems leading to molecules retaining a biological activity of the compound.

The term "enantiomer" refers to isolated optically pure enantiomers, as opposed to a mixture (at any relative ratio) of isomers. Compounds for use in the invention may thus be optically pure enantiomers or any mixtures (e.g., racemate) thereof.

### Compositions

The compounds for use according to the invention may be formulated with any pharmaceutically acceptable excipient, vehicle or carrier. They may be in the form of ointment, gel, paste, liquid solutions, suspensions, tablets, gelatin capsules, capsules, suppository, powders, nasal drops, or aerosol, preferably in the form of an injectible solution or suspension.

For injections, the compounds are generally packaged in the form of liquid suspensions, which may be injected e.g. via syringes or perfusions. In this respect, the compounds are generally dissolved in saline, physiological, isotonic or buffered solutions, compatible with pharmaceutical use and known to the person skilled in the art. Thus, the compositions may contain one or more agents or excipients selected from dispersants, solubilizers, stabilizers, preservatives, etc. Agents or excipients that can be used in liquid and/or injectable formulations are notably methylcellulose, hydroxymethylcellulose, carboxymethylcellulose, polysorbate 80, mannitol, gelatin, lactose, vegetable oils, acacia, etc. The carrier can also be selected, for example, from methyl-beta-cyclodextrin, a polymer of acrylic acid (such as carbopol), a mixture of polyethylene glycol and polypropylene glycol, monoetrhanol amine and hydroxymethyl cellulose.

The compounds for use according to the invention may be administered by injection, preferably by intramuscular, subcutaneous, transdermal, intraveinous or intraarterial injection; or by nasal, oral, mucosal or rectal administration; or by inhalation. The compounds for use according to the invention may also be administered repeatedly to the subject.

The compositions generally comprise an amount of a compound of formula A that is effective to inhibit sPLA2GIB.

The compositions according to the invention can comprise from about 0.01 µg to 1000 mg of a compound of formula A, for example between 0.05 µg and 600 mg, preferably between 0.05µg and 500 mg, for example between 5 mg and 500 mg. The dosages may be adjusted by the skilled person depending on the disease and subject. A particular dosage for compound T is comprised between 50 mg and 500 mg.

The compositions of the invention can further comprise one or more additional active compounds, for simultaneous, separate or sequential use.

In a particular embodiment, azole compounds of the invention of formula A, may be used in combination with other sPLA2GIB inhibitors, such as for instance anti-sPLA2GIB antibodies or derivatives thereof, inhibitory nucleic acids, peptides, small drugs and soluble receptors. Examples of such compunds are disclosed in WO2015/097140, WO2017/037041 or WO2017/060405.

In another embodiment, the azole compounds of formula A may be used in combination with one or more further active agents such as anti-viral agent(s) (e.g., anti-retroviral agents) or anti-cancer agent(s).

The invention also relates to a pharmaceutical composition comprising a compound of formula A as described herein; and an additional sPLA2GIB inhibitor selected from the group consisting of anti-sPLA2GIB antibodies or derivatives thereof, inhibitory nucleic acids, peptides, small drugs and soluble receptors; and, optionally, a pharmaceutically acceptable excipient. The most preferred additional sPLA2GIB inhibitors are anti-sPLA2GIB antibodies (as disclosed, for example, in WO2019/166664 or WO2019/166665).

The invention also relates to a pharmaceutical composition comprising a compound of formula A as described herein, in combination with any antiviral agent known in the art, or any anti-cancer agent known in the art; and, optionally, a pharmaceutically acceptable excipient.

The compositions of the invention may be formulated into any suitable device or container such as syringe, ampoule, flask, bottle, pouch, etc.

The invention also relates to a kit comprising (i) a container comprising a compound of formula A as described herein, (ii) a container comprising a further sPLA2GIB inhibitor and/or an anti-viral agent or anticancer agent, as described herein, and optionally (iii) written instructions for using the kit.

### Diseases

The compounds and compositions of the invention may be used to treat any disease related to an inappropriate (e.g., defective or improper) immune response, particularly to an inappropriate CD4 T cell activity, as well as any disease where an increased immunity may ameliorate the subject condition. These diseases are sometime referred to as "immune disorders" or "immunodeficiencies" in the present application. This includes particularly immunodefective situations caused by infections (such as viral or bacterial infections) or cancers.

As used herein, the term "treatment" or "treat" refers for instance to any clinical intervention in an attempt to alter the natural course of the subject being treated, and can be performed either for preventive or curative purpose. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, compositions and methods of the invention are used to delay development of a disease or disorder or to slow the progression of a disease or disorder.

Examples of diseases that can benefit from such treatment are all diseases with an immunodeficiency such as HIV-mediated immunodeficiency. In this regard, in a particular embodiment, the invention is directed to methods for treating an immunodeficiency or an associated disorder in a subject in need thereof, comprising administering a compound of formula A to said subject.

In another particular embodiment, the invention is directed to a compound of formula A for use for treating an immunodeficiency or an associated disorder in a subject in need thereof.

Immunodeficiencies and associated disorders designate any condition or pathology characterized by and/or caused by a reduced immune function or response in a subject. Immunodeficiencies may be caused by e.g., viral infection (e.g., HIV, hepatitis B, etc.), bacterial infection, cancer, or other pathological conditions. The terme "immunodeficiency-associated disorder" therefore designates any disease caused by or associated with an immunodeficiency. The invention is particularly suitable for treating immunodeficiencies related to CD4-T cells, and associated diseases.

In a particular embodiment, azole compounds of the invention are used for treating infectious dieseases such as viral diseases. More particularly, they may be used for treating AIDS in a HIV-infected subject in order to restore CD4 T cell function in a HIV-infected subject, and suppress or reverse HIV-mediated immunodeficiency.

In a particular embodiment, the invention relates to methods of treating HIV infection in a subject by administering a compound of formula A to said subject. In some embodiments, the subject is an early HIV patient and the methods results in increasing the probability that the patient is a HIV controller. In some embodiments the subject is a patient with low immunoreconstitution after antiretroviral treatment and/or with severe idiopatic CD4 T lymphopenia (ICL). The invention also relates to a method for increasing CD4-T cell activity in a HIV-infected subject by administering a compound of formula A to said subject.

The invention may be used to treat subjects at an early stage of the infection, to prevent or reduce occurrence, extent, or duration of an immunodeficiency. Typically, they can be administered immediatety upon detection of an infectious disease, and prior to appearance of clinical signs.

Administered very early during infection by HIV, compounds of formula A can prevent evolution towards an immune disease and prevent appearance of the immune deficiency, and they can lead the patients toward a HIV controller status.

The compounds of the invention may also be administered later in infected subjects, either alone or in combination with other active agents such as antiviral agent(s), in order to facilitate the recovery of a functional immune system leading to a control of HIV infection by the immune system. In such regimen, they accelerate especially the recovery of CD4 T lymphocytes and the restoration of their functions. Accordingly, in a particular embodiment, the invention comprises simultaneously, separately or sequentially administering to subject having a viral infection (i) a compound of formula A, and (ii) an antiviral agent. Such protocol is particularly suited for treating HIV infected subjects, wherein compound A is used in combination with antiretroviral therapy (e.g., HAART), allowing to reduce HAART or even to at least temporarily interrupt HAART treatment which is known for its severe detrimental effects.

The invention also provides methods for treating cancer by increasing an immune response in the subject, comprising administering a compound of formula A to said subject. The invention also provides methods of treating CD4 T cell-linked immunodeficiency associated with cancer in a subject by administering a compound of formula A to said subject.

The duration, dosages and frequency of administering compounds or compositions of the invention may be adapted according to the subject and disease. The treatment may be used alone or in combination with other active ingredients, e.g., additional sPLA2 inhibitors, either simultaneously or separately or sequentially.

The compounds or compositions according to the invention may be administered in various ways or routes such as, without limitation, by systemic injection, intramuscular, intravenous, intraperitoneal, cutaneous, subcutaneous, dermic, transdermic, intrathecal, ocular (for example corneal) or rectal way, or by a topic administration on an inflammation site, and preferably by intramuscular or intravenous injection.

A typical regimen comprises a single or repeated administration of an effective amount of a compound of formula A over a period of one or several days, up to one year, and including between one week and about six months. It is understood that the dosage of a pharmaceutical compound or composition of the invention administered *in vivo* will be dependent upon the age, health, sex, and weight of the recipient (subject), kind of concurrent treatment, if any, frequency of treatment, and the nature of the pharmaceutical effect desired. The ranges of effectives doses provided herein are not intended to be limiting and represent preferred dose ranges. However, the most preferred dosage will be tailored to the individual subject, as is understood and determinable by one skilled in the relevant arts (see, e.g., Berkowet et al., eds., The Merck Manual, 16th edition, Merck and Co., Rahway, N.J., 1992; Goodmanetna., eds., Goodman and Cilman's The pharmacological Basis of Therapeutics, 10th edition, Pergamon Press, Inc., Elmsford, N.Y., (2001)).

Further aspects and advantages of the invention are disclosed in the following experimental section, which shall be considered as illustrative.

### EXAMPLES

### Example 1: Screening small inhibitory molecules of sPLA2hGIB

The inventors have tested different series of small chemical molecules (99 molecules in total) for their ability to inhibit human sPLA2hGIB.

The inventors have characterized the inhibitory or non-inhibitory properties of these molecules with an enzyme inhibition test of sPLA2hGIB using tritiated *E. coli* membranes. (as previously described by Rouault et *al.,* 2006).

### Results

In this test, 98 of the 99 pre-selected molecules did not inhibit phospholipase sPLA2hGIB (Fig. 1). Only compound Terconazole (compound T), which is a triazole-family amphiphile cationic agent, significantly inhibited sPLA2hGIB using the AteroDX® test (Aterovax), as shown in Figure 1 (compound number #77). Indeed, Terconazole is the only compound that causes a decrease in sPLA2hGIB specific enzymatic activity greater than 15 % at 100 µM. More specifically, Terconazole inhibits sPLA2hGIB activity by more than 30%, approximately 33 %.

### Example 2: Inhibition of the enzymatic activity of sPLA2hGIB by Terconazole

The inhibitory activity of Terconazole on sPLAhGIB was further confirmed according to the method by Rouault et al., 2006, as demonstrated in Figure 2A.

In this experiment, Terconazole compound (PRESTWICK CHEMICAL, Illkirch-Graffenstaden) was used at different concentrations, i.e., 10 µM, 30 µM, 100 µM and 300 µM. The condition 'no hGIB' (0%) represents the non-specific hydrolysis of phospholipids present in *E. coli* membranes radio-labeled with [³H]oleate. The condition 'DMSO' represents hGIB activity alone with DMSO 1 % (the solvent used to dissolve Terconazole).

Results shown in Figure 2A demonstrate that Terconazole inhibits sPLA2hGIB, with an IC50 of 100-150 µM. Figure 2A also shows that the inhibition of sPLA2GIB by Terconazole compound is dose-dependent.

### Example 3: Inhibition of the enzymatic activity of several different sPLA2 phospholipases (i.e., sPLA2hGIB, sPLA2hGIIA, sPLA2hGV and sPLA2hGX) by Terconazole

In this experiment, the effect of Terconazole compound (PRESTWICK CHEMICAL, Illkirch-Graffenstaden) has been tested on different sPLA2 phospholipases, under the same conditions as those described above in Example 2 (according to the method by Rouault et al., 2006). The following concentrations of Terconazole were used: 3 µM, 10 µM, 30 µM, 100 µM and 300 µM.

Results shown in Figure 2B demonstrate that Terconazole inhibits sPLAhGIB and also other sPLA2 phospholipases sPLA2hGIIA, sPLA2hGV and sPLA2hGX, in a dose-dependent manner, although with a lower efficacy.

More particularly, Figure 2B shows that sPLA2hGX phospholipase is inhibited with an IC50 close to 150µM, and sPLA2hGIIA and sPLA2hGV phospholipases are inhibited with IC50 greater than 300µM.

### Example 4: Competition of Terconazole with the binding of sPLA2hGIB to mPLA2R1

The inventors have developed and used an AlphaLISA®-based test (Figure 3A) capable of detecting binding between the soluble form of mPLA2R1 (which includes the whole extracellular part), and sPLA2hGIB. In this test, it is possible to introduce other molecules in order to study the competition between the different molecules.

Figure 3A shows a general principle of the measurement of sPLA2 of interest: binding to the whole extracellular part of HA-tagged mPLA2R1 is measured using AlphaLISA® technology. HA-tagged receptors are in the presence of sPLA2 of interest, then in the presence of a specific biotinylated anti-sPLA2 antibody; "acceptor" beads are coupled to an anti-HA antibody (PerkinElmer) and "donor" beads are coupled to Streptavidine (PerkinElmer). The binding of a HA-tagged receptor and a sPLA2 of interest in solution, results in a proximity of donor and acceptor beads, allowing for energy transfer and the production of a light signal, which is then measured.

In order to better understand the specificity of Terconazole inhibition, the inventors tested the effect of Terconazole on the binding between sPLA2hGIB and its receptor called PLA2R1, using the AlphaLISA®-based test according to Figure 3A.

As shown Figure 3B, Terconazole (compound T) decreased the binding between mPLA2R1 receptor and sPLA2hGIB (with IC50 close to 100 µM).

This example also showed that Terconazole is a dose-dependent inhibitor of sPLA2hGIB enzymatic activity.

In addition, Terconazole very significantly inhibits the binding of mPLA2R1 to sPLA2hGIB, confirming the inhibitor acts through the active site of sPLA2GIB.

### REFERENCES

Lambeau G and Gelb MH (2008) Biochemistry and physiology of mammalian secreted phospholipases A2. Annu Rev Biochem. 77:495-520*.*
Rouault et al. (2006) Neurotoxicity and other pharmacological activities of the snake venom phospholipase A2 OS2: the N-terminal region is more important than enzymatic activity. Biochemistry 45, 18:5800-5816.

## Claims

1. A compound of formula A 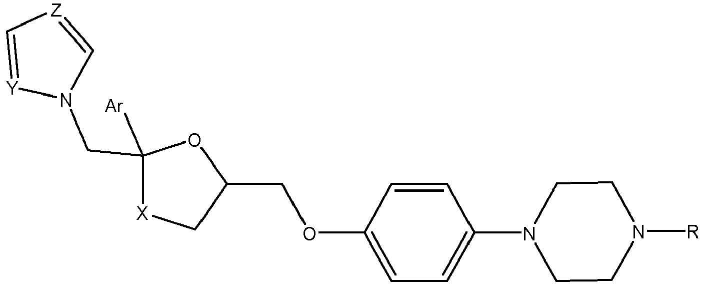 wherein:
• Ar is an aryl group, optionally substituted by one or more halogen atoms;
• X is CH2 or O (oxygen atom);
• Y and Z both are respectively:
either N (nitrogen atom) and N (nitrogen atom),
or CH or N (nitrogen atom);
• R is a -COCH3, -CH(CH3)2, 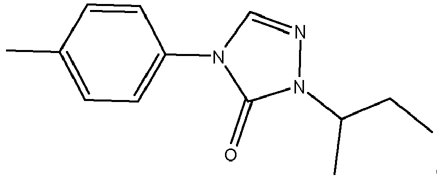 or 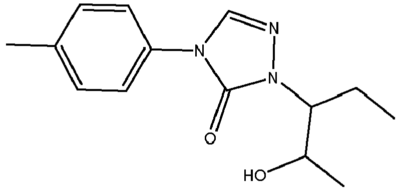 group;
or a salt, ester, hydrate, racemate, enantiomer (e.g. R or S form), prodrug or metabolite thereof,
for use to induce or stimulate an immune response or for treating an immunodeficiency disorder in a subject in need thereof.

2. The compound for use according to claim 1, wherein Ar is a phenyl group substituted by one or two halogen atoms, preferably chlorine or fluorine.

3. The compound for use of claim 1 or 2, wherein Y and Z are both nitrogen atoms.

4. The compound for use of any one of claims 1 to 3, wherein X is an oxygen atom.

5. The compound for use of claim 1, wherein the compound is terconazole, itraconazole, posaconazole or ketoconazole.

6. The compound for use of any one of the preceding claims, wherein said compound inhibits sPLA2GIB.

7. The compound for use of any one of the preceding claims, wherein said compound is combined with another sPLA2GIB inhibitor selected from the group consisting of antibodies or derivatives thereof, inhibitory nucleic acids, peptides, small drugs and soluble receptors.

8. The compound of any one of any one of the preceding claims, for use to induce or stimulate CD4 T cells in the subject.

9. The compound for use of any one of the preceding claims, wherein the subject has an immune disorder, an infectious disease (such as a viral or bacterial disease) or a cancer.

10. The compound for use of any one of the preceding claims, wherein the compound is administered to the subject at a dose comprised between 0.01 µg and 1000 mg.

11. The compound for use of any one of the preceding claims, wherein the compound is administered repeatedly to the subject.

12. The compound for use of any one of the preceding claims, wherein the compound is administered by injection, preferably by intramuscular, subcutaneous, transdermal, intraveinous or intraarterial injection; or by nasal, oral, mucosal or rectal administration; or by inhalation.

13. The compound for use of any one of the preceding claims, wherein the compound is formulated in a pharmaceutical composition comprising a pharmaceutically acceptable carrier or excipient.

14. The compound for use of any one of the preceding claims, wherein the subject is a human subject.

15. A pharmaceutical composition comprising: (i) a compound of formula A as defined in any one of claims 1 to 5, and (ii) another sPLA2GIB inhibitor selected from the group consisting of antibodies or derivatives thereof, inhibitory nucleic acids, peptides, small drugs and soluble receptors; for combined, separate, or sequential administration.

16. A pharmaceutical composition comprising: (i) a compound of formula A as defined in anyone of claims 1 to 5, and (ii) an antiviral or anti-cancer agent; for combined, separate, or sequential administration.
